(19) Europäisches Patentamt
European Patent Office
Office européen des brevets

(11) **EP 2 506 586 A2**

(12) **EUROPEAN PATENT APPLICATION**

(43) Date of publication:
**03.10.2012 Bulletin 2012/40**

(51) Int Cl.:
*H04N 13/04* (2006.01)   *H04N 13/00* (2006.01)

(21) Application number: **12157124.4**

(22) Date of filing: **27.02.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **31.03.2011 JP 2011079926**
**01.08.2011 JP 2011168616**

(71) Applicant: **Fujifilm Corporation**
**Minato-ku**
**Tokyo (JP)**

(72) Inventors:
• **Kanagawa, Akiko**
**Kanagawa (JP)**

• **Kaneko, Yasuhiko**
**Kanagawa (JP)**
• **Hiruoka, Jun**
**Kanagawa (JP)**
• **Sakaguchi, Yuichi**
**Kanagawa (JP)**
• **Mochizuki, Naoki**
**Kanagawa (JP)**
• **Ida, Noriaki**
**Kanagawa (JP)**

(74) Representative: **Klunker . Schmitt-Nilson . Hirsch**
**Patentanwälte**
**Destouchesstrasse 68**
**80796 München (DE)**

(54) **Stereoscopic display apparatus**

(57)    A stereoscopic display apparatus that performs a stereoscopic display using a right-eye image and a left-eye image acquired by radiographing a subject plural times while changing an incidence angle of radiation on the subject, includes a display unit that stereoscopically displays the right-eye image and the left-eye image, and changing means that shifts at least one of the right-eye image and the left-eye image displayed on the display unit to change an inter-image distance being a distance between the right-eye image and the left-eye image in a direction parallel to a straight line connecting the right eye and the left eye of an observer.

FIG. 1

EP 2 506 586 A2

**Description**

BACKGROUND OF THE INVENTION

This invention relates to a stereoscopic display apparatus that performs a stereoscopic display using a right-eye image and a left-eye image acquired through radiography (stereoscopic photography).

Recently, medical image-related network systems such as PACS (Picture Archiving and Communication Systems) have been developed and opportunities for storing radiographic images during diagnosis as digital data in a server and using the digital data for follow-up later or transmitting the radiographic images during the previous diagnosis as image data for use by other remote hospitals is increasing.

In radiography, the invention of a stereoscopic display apparatus of a radiographic image that irradiates a subject with radiation from different angles, that acquires (stereoscopically photographs) a right-eye image and a left-eye image, and that performs a stereoscopic display using the acquired images is known.

In fluoroscopic images such as radiographic images, it is difficult to determine which part of an image is located in the foreground, discernment being difficult in the depth direction. Therefore, when a radiographic image is stereoscopically displayed, there is a merit that it is easy to determine the three-dimensional distribution of pathological changes or the like (see JP 2010-200787 A and JP 3780217 B).

SUMMARY OF THE INVENTION

As described above, when a radiographic image is stereoscopically displayed, it is easy to determine locations in the depth direction. However, for example, when a stereoscopic display providing an excessively-protruding view is observed, this can increase eye fatigue or even cause erroneous diagnosis in some cases.

Therefore, in a stereoscopic display providing an excessively-protruding view, it would be preferable if the position could be changed in the depth direction.

An object of the present invention is to provide a stereoscopic display apparatus which can simply change the depth of a stereoscopic display in a right-eye image and a left-eye image radiographed (stereoscopically photographed) for the purpose of a stereoscopic display.

In order to achieve the above-mentioned objects, the resent invention provides a stereoscopic display apparatus that performs a stereoscopic display using a right-eye image and a left-eye image acquired by radiographing a subject plural times while changing an incidence angle of radiation on the subject, comprising:

a display unit that stereoscopically displays the right-eye image and the left-eye image; and
changing means that shifts at least one of the right-eye image and the left-eye image displayed on the display unit to change a distance (an inter-image distance) between the right-eye image and the left-eye image in a direction parallel to a straight line connecting the right eye and the left eye of an observer.

Further, preferably, the inter-image distance includes a first inter-image distance and a second inter-image distance,
the first inter-image distance being calculated on the basis of radiographic conditions of the right-eye image and the left-eye image, and
the second inter-image distance being calculated on the basis of the radiographic conditions, and observation conditions, display conditions and image processing conditions of the stereoscopic display, and
wherein the range of the inter-image distance which can be changed by the changing means is calculated on the basis of the first inter-image distance and the second inter-image distance.

Further comprising warning means that gives a warning when the inter-image distance is out of the changeable range of the inter-image distance by the changing means.

Further, preferably, the warning of the warning means includes at least one of stopping the changing of the inter-image distance and displaying the purport thereof.

Further, preferably, wherein when the radiographic conditions includes a radiation source-image reception plane distance (SID) D, an irradiation angle of a radiation source (a rotation angle of a radiation source arm) $\theta_1$ and $\theta_2$ ($\theta_1 \leq \theta_2$), a pressing thickness (a radiographic stand-pressing plate surface distance) $l_p$, and a case thickness (a radiographic stand-image reception plane distance) $l_c$, the range of the first inter-image distance $x_m$ is expressed by:

$$\frac{l_c \cdot D\sin\theta_2 \cdot (D\cos\theta_1 - l_c) - l_c \cdot D\sin\theta_1 \cdot (D\cos\theta_2 - l_c)}{(D\cos\theta_1 - l_c)(D\cos\theta_2 - l_c)} \leqq x_m \leqq$$

$$\frac{l_p \cdot D\sin\theta_2 \cdot (D\cos\theta_1 - l_p) - l_p \cdot D\sin\theta_1 \cdot (D\cos\theta_2 - l_p)}{(D\cos\theta_1 - l_p)(D\cos\theta_2 - l_p)} \quad \cdots (4)$$

.

Further, preferably, when the observation conditions include an observation distance VD which is a distance between the display unit and the observer, a binocular distance $D_{eye}$, an imaging plane distance x, a parallax angle $\phi_2$ (a difference between a binocular angle $\beta$ in the display unit and a binocular angle $\alpha$ at an imaging position), and a right-left parallax d' on the display unit, the right-left parallax d' is expressed by

$$d' = \frac{\tan\frac{\phi_2}{2} \cdot (D_{eye}^2 + 4VD^2)}{2VD + D_{eye} \cdot \tan\frac{\phi_2}{2}} \quad \cdots (15)$$

Further, preferably, the display conditions include an enlargement and reduction ratio based on an inter-pixel distance (pixel size) of the display unit.

Further, preferably, the image processing conditions include a display magnification of the right-eye image and the left-eye image.

Further, preferably, when the display conditions include an enlargement and reduction ratio $m_1$ and the image processing conditions include a display magnification $m_2$, the range of the second inter-image distance $\Delta d$ is expressed by

$$\Delta d = \frac{d'}{m_1 m_2} - x_m \quad \cdots (18)$$

,

and

wherein when the difference in rotation angle of the radiation source arm is $\psi_1$ ($\psi_1 = \theta_2 - \theta_1$), the second inter-image distance $\Delta d$ is expressed by

$$\frac{\tan\frac{\phi_2}{2} \cdot (D_{eye}^2 + 4VD^2)}{m_1 m_2 (2VD + D_{eye} \cdot \tan\frac{\phi_2}{2})} - \frac{2 \cdot D \cdot \tan\frac{\phi_1}{2} \cdot l_p}{D - l_p} \leqq \Delta d \leqq$$

$$\frac{\tan\frac{\phi_2}{2} \cdot (D_{eye}^2 + 4VD^2)}{m_1 m_2 (2VD + D_{eye} \cdot \tan\frac{\phi_2}{2})} - \frac{2 \cdot D \cdot \tan\frac{\phi_1}{2} \cdot l_c}{D - l_c} \quad \cdots (19)$$

.

Further comprising an image difference display area in which the inter-image distance between the right-eye image and the left-eye image is displayed by the use of thumbnail images,
wherein the changing means changes the distance between the thumbnail images displayed in the image difference display area to adjust the depth of the stereoscopic display.

Further, preferably, the changing means has an automatic control function, and
wherein the automatic control function is to automatically change the inter-image distance depending on the radiographic conditions.

Further, preferably, the automatic control function is to change the inter-image distance to any one of a maximum value, a median value, and a minimum value of the range of the changeable inter-image distance.

Further comprising an image server that stores a plurality of the inter-image distances between right-eye images and left-eye images which can be stereoscopically displayed,
wherein the changing means has an automatic control function, and
wherein the automatic control function is to automatically change the inter-image distance of the present stereoscopic display depending on the inter-image distance of the other stereoscopic displays.

Further, preferably, blanks of the right-eye image and the left-eye image formed by causing the changing means to change the inter-image distance are painted with black.

According to the present invention, since the depth of a stereoscopic display can be freely changed in the range in which the stereoscopic view is not destroyed, it is possible to reduce the burden on the eyes when viewing the stereoscopic display, and thus to prevent erroneous diagnosis due to the eye fatigue.

BRIEF DESCRIPTION OF THE DRAWINGS

FIG. 1 is an overview diagram illustrating an example of a stereoscopic display apparatus according to the present invention.
FIG. 2 is a block diagram illustrating an example of the system configuration of the stereoscopic display apparatus according to the present invention.
FIG. 3 is a diagram illustrating an example of the display screen of the stereoscopic display apparatus according to the present invention.
FIG. 4 is a diagram illustrating a variation in depth and appearance from a right eye and a left eye.
FIG. 5A is a diagram illustrating depth and appearance in a stereoscopic display and FIG. 5B is a diagram illustrating the right-eye image and left-eye image at that time.
FIG. 6A is a diagram illustrating the right-eye image and the left-eye image when an inter-image distance which is a distance between the right-eye image and the left-eye image is changed and FIG. 6B is a diagram illustrating depth and appearance in a stereoscopic display in this case.
FIG. 7 is a diagram illustrating a method of calculating the range of an inter-image distance (a first inter-image distance) providing the maximum value and the minimum value of the depth of a stereoscopic display according to a first embodiment of the present invention in mammography.
FIG. 8 is a flowchart illustrating the flow of processes when the depth of the stereoscopic display is changed in the first embodiment of the present invention.
FIG. 9 is a diagram illustrating depth and appearance in a stereoscopic display when observation conditions are considered in a second embodiment of the present invention.
FIG. 10 is a flowchart illustrating the flow of processes when the depth of the stereoscopic display is changed in the second embodiment of the present invention.

DETAILED DESCRIPTION OF THE INVENTION

A stereoscopic display apparatus according to the present invention will be described below in detail with reference to exemplary embodiments shown in the accompanying drawings.

First Embodiment

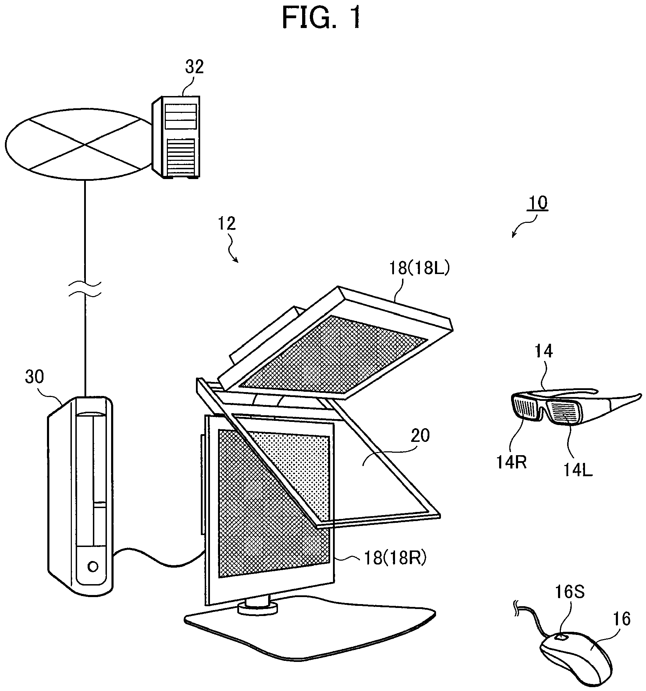

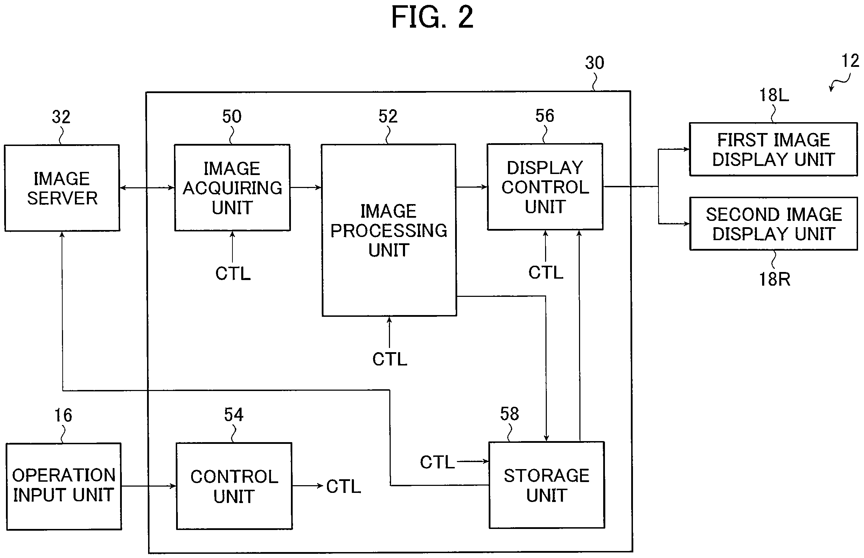

FIG. 1 is an overview diagram illustrating the entire configuration of an example of a stereoscopic display apparatus according to the present invention. FIG. 2 is a block diagram illustrating an example of the system configuration of the stereoscopic display apparatus according to the present invention.

The stereoscopic display apparatus 10 according to the present invention includes a display unit 12, a pair of polarizing glasses 14, an operation input unit 16, and a console 30.

The display unit 12 includes a first image display unit 18L, a second image display unit 18R, and a beam splitter mirror 20. The display unit 12 performs a stereoscopic display by displaying a left-eye image on the first image display unit 18L and displaying a right-eye image on the second image display unit 18R, and may also display a normal planar display by displaying the same image on the first image display unit 18L and the second image display unit 18R.

In the first embodiment, it is assumed that the display unit 12 is observed from an observation position at a predetermined distance from the display unit 12 (predetermined observation conditions) and that an inter-pixel distance (pixel size) of the display unit 12 (the first image display unit 18L and the second image display unit 18R) and an enlargement and reduction ratio are set to predetermined value (predetermined display conditions and image processing conditions).

By causing an observer to observe the display unit 12 from the above-mentioned observation position by the use of a pair of polarizing glasses 14 to be described later, it is possible to achieve a stereoscopic display based on a right-eye image and a left-eye image.

The stereoscopic display apparatus 10 according to the present invention is connected, for example, to an image network system such as PACS via an image acquiring unit 50 to be described later of the console 30 as described above and acquires a right-eye image and a left-eye image allowing a stereoscopic view in a radiographic image from an image server 32 included in the image network system. The stereoscopic display apparatus 10 may be connected directly to a radiographic imaging system or the like and may acquire a right-eye image and a left-eye image allowing a stereoscopic view.

The right-eye image and the left-eye image allowing a stereoscopic view are a pair of radiographic images radiographed (stereoscopically photographed) (radiographed under predetermined radiographic conditions) in a state where the irradiation position of radiation, that is, a bulb (radiation source) position, is shifted in a predetermined direction by a predetermined distance in a radiographic imaging apparatus or the like.

The right-eye image and the left-eye image allowing a stereoscopic view may be a pair of radiographic images radiographed in a state where the irradiation direction of the bulb is shifted or may be a pair of radiographic images radiographed in a state where a subject rotates and an incidence angle of radiation on a subject is changed.

The acquired right-eye image and left-eye image are subjected to a predetermined image process by an image processing unit 52 to be described later of the console 30 and are displayed on the first image display unit 18L and the second image display unit 18R, respectively, by a display control unit 56 to be described later.

Optical filters which are not shown in the drawings and which polarize light emitted from the display units in predetermined different directions may be disposed in front of the first image display unit 18L and the second image display unit 18R.

Here, the first light of the left-eye image displayed on the first image display unit 18L is polarized in a predetermined direction by the optical filter not shown and is reflected by the beam splitter mirror 20.

Similarly, the second light of the right-eye image displayed on the second image display unit 18R is polarized in a predetermined direction different from that of the first light by the optical filter not shown and is transmitted by the beam splitter mirror 20.

Therefore, the reflected first light and the transmitted second light are changed to combined light having different polarization directions and traveling in the same direction by the beam splitter mirror 20.

In the left-eye image displayed on the first image display unit 18L, when it is reflected by the beam splitter mirror 20, the first light is vertically inverted (exactly, the front and rear sides in the traveling direction of the light are inverted), and it is thus necessary to vertically invert a display image in advance. Therefore, the image processing unit 52 to be described later of the console 30 performs a process of vertically inverting an image on the left-eye image displayed on the first image display unit 18L in advance, in addition to a process of changing an enlargement and reduction ratio.

The angle of the beam splitter mirror 20 is adjusted and fixed so that the left-eye image displayed on the first image display unit 18L and the right-eye image displayed on the second image display unit 18R overlap with each other when an operator (an observer) at the observation position watches the display unit 12 of the stereoscopic display apparatus 10 from the front side.

The pair of polarizing glasses 14 transmits the first light from the first image display unit 18L through the use of a left-eye polarizing lens 14L, transmits the second light from the second image display unit 18R through the use of a right-eye polarizing lens 14R, and blocks the other light.

Therefore, the left-eye image displayed on the first image display unit 18L is recognized by the left eye of the operator wearing the pair of polarizing glasses 14 and the right-eye image displayed on the second image display unit 18R is recognized by the right eye of the operator.

When recognizing images having parallax by the sue of the left eye and the right eye, a human being recognizes the image as a stereoscopic display. Accordingly, the operator wearing the pair of polarizing glasses 14 observes a stereoscopic display based on the right-eye and the left-eye image by simultaneously recognizing the right-eye image and the left-eye image having parallax by the sue of the right eye and the left eye.

The operation input unit 16 may be, for example, a pointing device such as a mouse used for operating a computer and cursor 24 to be described later on the display screen recognized by the operator can be freely operated by operating the operation input unit 16. The operation input unit 16 is not limited to the mouse, but may further include operation input means such as a keyboard for inputting information in addition to the mouse.

The cursor 24 to be described later is calculated in position and drawn by the use of the control unit 54 to be described later of the console 30 and is displayed on the first image display unit 18L and the second image display unit 18R through the use of the display control unit 56 to be described later.

As shown in FIG. 2, the console 30 includes an image acquiring unit 50, an image processing unit 52, a control unit 54, a display control unit 56, and a storage unit 58. The console 30 is specifically constructed by a computer including a CPU (Central Processing Unit), a RAM (Random Access Memory), and a hard disk and the CPU, the RAM, the hard disk, and the like constitute the units of the console 30 in cooperation with each other.

The image acquiring unit 50 acquires image data of a right-eye image and a left-eye image allowing a stereoscopic display from the image server 32 in response to the operation input unit 16 via the control unit 54.

The image processing unit 52 performs a predetermined image process on the image data of the right-eye image and the left-eye image acquired by the image acquiring unit 50 and outputs image data which can be displayed on the first image display unit 18L and the second image display unit 18R. The image processing unit 52 performs an enlargement and reduction process using a predetermined an enlargement and reduction ratio on the right-eye image and the left-eye image on the basis of display software or the like. The enlargement and reduction process may be performed on the basis of information of the inter-pixel distance of the display unit 12 (the first image display unit 18L and the second image display unit 18R). As described above, an image process is performed on the left-eye image to be displayed on the first image display unit 18L so as to display a vertically-inverted image.

The image processing unit 52 may output the image data of the right-eye image and the left-eye image having been subjected to the image process to the storage unit 58 in response to an instruction from the control unit 54.

Information of the enlargement and reduction ratio of an image can be set by the use of the operation input unit 16. The image processing unit 52 may enlarge and reduce the right-eye image and the left-eye image on the basis of the information of the set enlargement and reduction ratio of an image.

The control unit 54 controls the operations of the image acquiring unit 50, the image processing unit 52, the display control unit 56, and the storage unit 58 in accordance with an instruction from the operation input unit 16 and displays the cursor 24 operated by the use of the operation input unit 16 on both images of the right-eye image and the left-eye image through the use of the display control unit 56.

The control unit 54 calculates the movable range in the depth direction by changing an image difference (inter-image distance) between the right-eye image and the left-eye image through the use of the image processing unit 52 and the display control unit 56 to be described later or using radiographic conditions.

The display control unit 56 displays the right-eye image and the left-eye image, which has been subjected to a predetermined image process by the image processing unit 52 and can be displayed, on the first image display unit 18L and the second image display unit 18R in response to an instruction from the control unit 54, and the control unit 54 displays the cursor 24, which is calculated in position and drawn, on the first image display unit 18L and the second image display unit 18R.

The display control unit 56 may read the image data of the right-eye image and the left-eye image stored in the storage unit 58 in response to an instruction from the control unit 54 and may display the image data on the first image display unit 18L and the second image display unit 18R.

The storage unit 58 stores the image data of the right-eye image and the left-eye image having been subjected to the predetermined image process by the image processing unit 52 and outputs the image data to the display control unit 56, if necessary, in response to the instruction from the control unit 54.

The storage unit 58 may output the image data of the stored right-eye image and left-eye image to the image server 32 in response to an instruction from the operation input unit 16 via the control unit 54.

The storage unit 58 may store radiographic conditions of the right-eye image and the left-eye image, the inter-image distance, the enlargement and reduction ratio of an image, and the like, in addition to the image data of the right-eye image and the left-eye image.

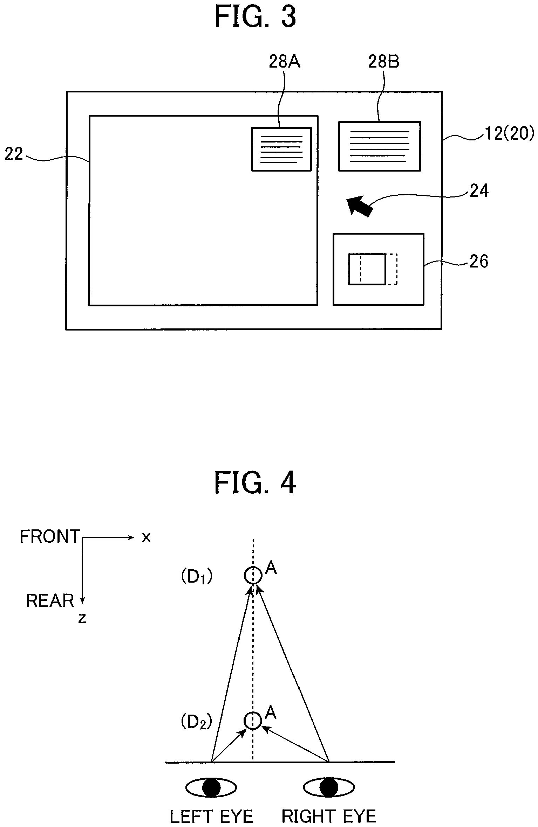

FIG. 3 is an example of a diagram illustrating a stereoscopic display screen recognized from the display unit 12 (the beam splitter mirror 20) in the stereoscopic display apparatus 10 according to the present invention.

As shown in FIG. 3, the stereoscopic display screen of the display unit 12 displays an image display area 22, a cursor 24, an image difference display area 26, and text display areas 28A and 28B.

The image display area 22 can perform a stereoscopic display and can also performs a planar display including a right-eye image and a left-eye image in response to an instruction input from the operation input unit 16 and in response to an input instruction (an input instruction to the text display areas 28A and 28B to be described later) to the picture (the display unit 12) based on the operation on the cursor 24.

The image display area 22 can simultaneously perform plural stereoscopic displays and may perform both the stereoscopic display and the planar display.

The cursor 24 gives an input instruction on the picture as described above and adjusts the stereoscopic display in the image display area in the depth direction by moving (shifting) the thumbnail images of the right-eye image and the left-eye image in the image difference display area 26 to be described later.

The image difference display area 26 displays by what the right-eye image and the left-eye image overlap separated from each other (an image difference (the inter-image distance) between the right-eye image and the left-eye image) in the stereoscopic display as a positional relationship between the thumbnail image of the left-eye image and the thumbnail image of the right-eye image.

The operator can confirm by what the right-eye image and the left-eye image are shifted from the original positions by confirming the image difference display area 26.

By operating the operation input unit 16 (dragging the mouse 16) to move the thumbnail image of the left-eye image and the thumbnail image of the right-eye image through the use of the cursor 24, it is possible to adjust the stereoscopic display in the depth direction.

Regarding the operation using the operation input unit 16, the movement of both thumbnail images, that is, the adjustment of the stereoscopic display in the depth direction, may be controlled, for example by rotating (scrolling) a wheel 16S of the mouse 16 forward and backward.

Regarding the movement of the thumbnail image, both the thumbnail image of the right-eye image and the thumbnail image of the left-eye image may be moved or only one thereof may be moved. The distance between the right-eye image and the left-eye image (the inter-image distance) in the stereoscopic display is changed depending on the distance between the thumbnail images.

Since the right-eye image and the left-eye image are images radiographed from different positions for the purpose of the stereoscopic display, an observer can recognize the stereoscopic display by displaying both images are displayed to overlap at the same position, causing the left eye to recognize the left-eye image, and causing the right eye to recognize the right-eye image. In the stereoscopic display apparatus 10 according to the present invention, the positional relationship among the first image display unit 18L, the second image display unit 18R, and the beam splitter mirror 20 is precisely adjusted in advance so as to display both images to overlap at the same position in the image display area 22 when the display unit is observed at the above-mentioned observation position.

When the display positions of the right-eye image and the left-eye image in the image display area 22 to be observed at the observation position are separated from each other at the first time of observation, the observer operates the operation input unit 16 to correct the display positions of the right-eye image and the left-eye image by software through the use of the display control unit 56, thereby performing a calibration so as to display both images to overlap at the same position.

In the stereoscopic display having been subjected to the calibration, the right-eye image and the left-eye image are displayed to overlap at the same position in the image display area 22 and the thumbnail image of the left-eye image and the thumbnail image of the right-eye image in the image difference display area 26 are displayed to overlap at the same position as described above.

In the image difference display area 26, frames or rectangles representing the right-eye image and the left-eye image may be displayed instead of the thumbnail images. This is because the image difference in the present stereoscopic display has only to be confirmed and has only to be finely adjusted in the depth direction.

Therefore, for example, the position of the present stereoscopic display may be represented by a dotted frame line, the left-eye image may be represented by a blue rectangle, and the right-eye image may be represented by a red rectangle. The rectangles may transmit the background and the overlapping area may be displayed with violet in which blue and red are superimposed.

When plural stereoscopic displays are displayed in the image display area 22, the thumbnail images of the stereoscopic display selected by the use of the cursor 24 are displayed in the image difference display area 26.

The text display areas 28A and 28B show menus, radiographing information of the right-eye image and the left-eye image, the inter-image distance between the right-eye image and the left-eye image, a warning from warning means to be described later, and the like. The text display area 28A in the image display area 22 can show a stereoscopic display and the text display area 28B other than the image display area 22 can show a planar display.

In the text display area 28A, similarly to the right-eye image and the left-eye image, and the stereoscopic display can be changed in the depth direction, the stereoscopic display may be changed in the depth direction by interlocking with the right-eye image and the left-eye image or may be changed in the depth direction independently of the right-eye image and the left-eye image.

Examples of the menus include switching between a stereoscopic display and a planar display in the image display area 22, selection of a display method, storage of a stereoscopic display (a right-eye image, a left-eye image, an inter-image distance therebetween), and storage of the inter-image distance, and the like.

The operator can point out the menus by the use of the cursor 24.

A method of displaying a single stereoscopic view in the image display area 22, a method of displaying plural stereoscopic views, a method of showing both a stereoscopic display and a planar display (at least one of a right-eye image and a left-eye image), and the like can be considered as a display method. These display methods can be selected in the menus.

The principle of the stereoscopic display in the present invention and the effect when a right-eye image (a viewpoint of a right eye) and a left-eye image (a viewpoint of a left eye) are shifted and displayed will be described below with reference to FIGS. 4 to 6B.

First, it is assumed in FIG. 4 that the right-left direction is defined as an X axis direction (where the right side is positive) and the front-rear direction is defined as a Z axis direction (where the rear side is positive). Then, when an object A located at the same position in the X axis direction moves away from an observer's viewpoint (when it goes apart in the Z axis direction (for example, moves from the position ($D_2$) in FIG. 4 to the position ($D_1$))), the object in the left-eye image appears to move to the left side and the object A in the right-eye image appears to move to the right side. When the object moves close to the observer's viewpoint (when it goes close in the Z axis direction (for example, moves from the position ($D_1$) in FIG. 4 to the position ($D_2$))), the object A in the left-eye image appears to move to the right side and the object A in the right-eye image appears to move to the left side.

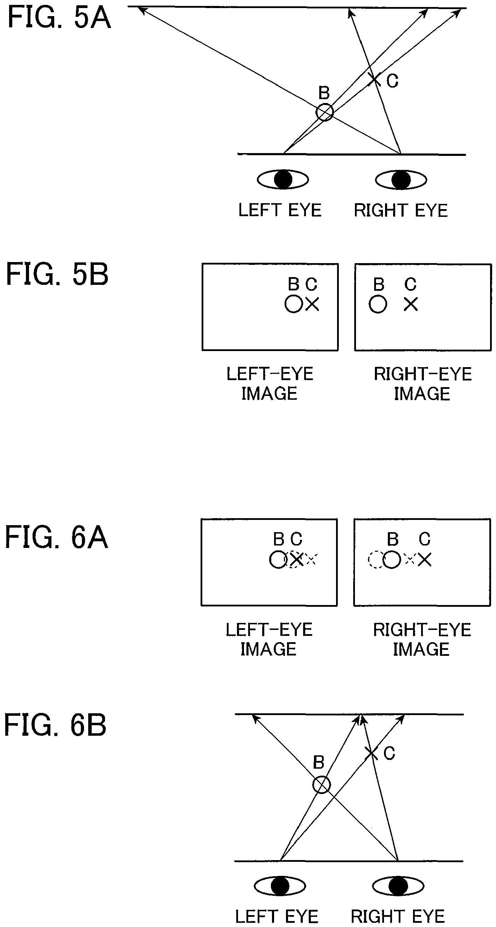

The effect when the right-eye image and the left-eye image are shifted and displayed will be described with reference to FIGS. 5A and 5B and FIGS. 6A and 6B on the basis of the fact shown in FIG. 4.

As shown in FIG. 5A, for example, when two objects B and C located spatially separated by a predetermined distance from each other are present relatively close to the observer's viewpoint, the right eye and the left eye recognize the objects as the right-eye image and the left-eye image shown in FIG. 5B, respectively.

The observer is made to view the right-eye image and the left-eye image including the objects B and C shown in FIG. 6A in which the left-eye image is parallel-shifted to the left side from FIG. 5B and the right-eye image is parallel-shifted to the right side from FIG. 5B (that is, the inter-image distance between the right-eye image and the left-eye image is made to increase). In the right-eye image and the left-eye image shown in FIG. 6A, the objects B and C are parallel-shifted from the position in FIG. 5B indicated by a dotted line to the position indicated by a solid line.

Then, as can be seen from the description with reference to FIG. 4, the observer's brain recognizes the objects B and C are located at the position shown in FIG. 6B deeper than in FIG. 5A.

Therefore, by shifting the right-eye image and the left-eye image shown in FIG. 5B in a predetermined direction (the moving direction of a radiation source (a radiographing direction), that is, the direction parallel to a straight line connecting the observer's right eye and left eye) and showing the observer to view them as the right-eye image and the left-eye image shown (indicated by the solid line) in FIG. 6A, the observer's recognition in the depth direction can be shifted.

That is, it is possible to adjust the stereoscopic display in the depth direction.

When the inter-image distance between the right-eye image and the left-eye image varies in the direction parallel to the straight line connecting the observer's right eye and left eye, the display appears to vary in the depth direction. Accordingly, even when the right-eye image and the left-eye image are slightly obliquely moved, the display is adjusted in the depth direction.

That is, the direction in which the right-eye image and the left-eye image are shifted does not have to be exactly parallel to the direction of the straight line connecting the observer's right eye and left eye, but both directions have only to be substantially parallel to each other so as to change the inter-image distance in the direction of the straight line.

The principle of the present invention and the effect when the right-eye image and the left-eye image are shifted and displayed in a predetermined direction are described hitherto.

Therefore, the stereoscopic display apparatus 10 according to the present invention shown in FIGS. 1 and 2 can cause the observer to recognize a deeper stereoscopic display by causing the image processing unit 52 of the console 30 to slightly shift the right-eye image and the left-eye image allowing a stereoscopic view to the right side and the left side, respectively, to form a new right-eye image and a new left-eye image and causing the observer to view the new right-eye image and the new left-eye image.

That is, the stereoscopic display apparatus 10 shown in FIGS. 1 and 2 can change the depth of a stereoscopic display by shifting the right-eye image and the left-eye image radiographed for a stereoscopic display in the direction (the horizontal right-left direction in this case) parallel to the straight line connecting the observer's right eye and left eye as described above.

The stereoscopic display apparatus 10 according to the present invention can change the depth of a stereoscopic display as described above, but the change of the depth is restricted. When the right-eye image and the left-eye image are displayed to be excessively separated from each other, the observer's brain recognizes the right-eye image and the left-eye image as different images and cannot thus obtain a stereoscopic view.

When the right-eye image and the left-eye image are exchanged in position, the observer's brain may recognize the stereoscopic display to reverse the depth positions.

When the stereoscopic view is destroyed in this way, it is not possible to perform a normal radiographic image diagnosis and there is a risk of causing an erroneous diagnosis when the depth positions are reversed. Therefore, in order not to cause such a phenomenon, it is necessary to predetermine the maximum value and the minimum value in the depth direction, that is, the range of the distance between the right-eye image and the left-eye image, and to give a warning when the shift is out of the range.

The calculation of the maximum value and the minimum value in the depth direction, that is, the range of the distance between the right-eye image and the left-eye image, will be described below with reference to mammography.

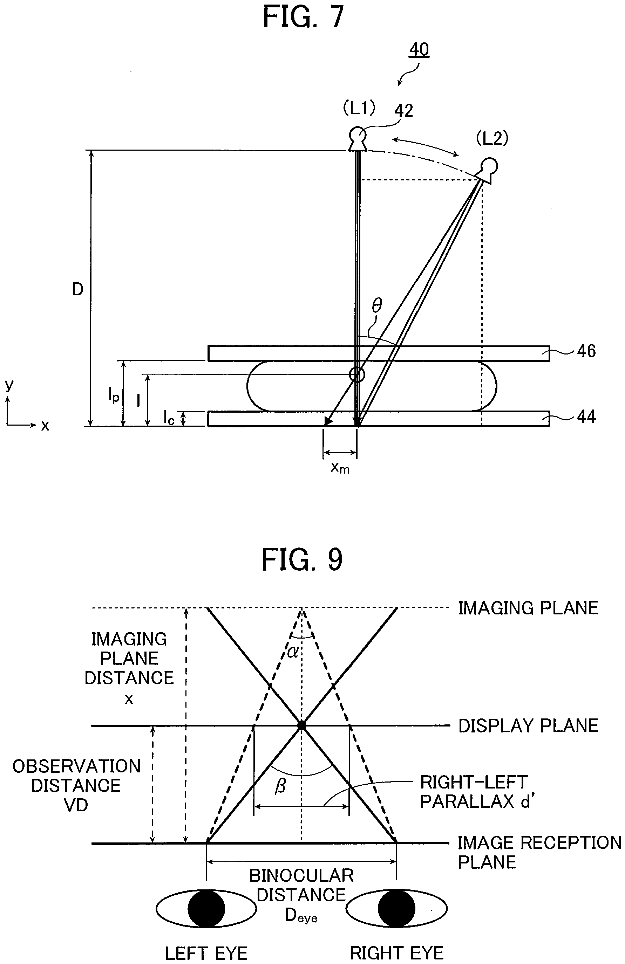

As shown in FIG. 7, a mammographic imaging apparatus 40 includes a radiation source 42 moving while maintaining a radiation source-image reception plane distance (SID) about the center of the image reception plane, a radiographic stand (case) 44 which can be adjusted in depth, and a pressing plate 46 pressing a patient's breast to the radiographic stand 44. A detector not shown and including the image reception plane is disposed below the radiographic stand 44.

When a stereoscopic display is performed in the mammography, a right-eye image and a left-eye image necessary for the stereoscopic display are acquired by mammographing (stereoscopically photographing) a subject from two places having different radiographic positions (radiation source positions).

In this case, the right-eye image and the left-eye image are captured at radiographic positions allowing a stereoscopic display by causing the stereoscopic display apparatus 10 to display both images as they are.

As shown in FIG. 7, a stereoscopic display is performed on the basis of images captured, for example, from two places in which the radiation source is located at an angle of 0° (L1) and the radiation source is located at an angle of θ (L2).

In the mammographic imaging apparatus 40, first, a radiation source-image reception plane distance (SID) is defined as D, a pressing thickness (a radiographic stand-pressing plate surface distance) is defined as $l_p$, a radiographic stand thickness (a radiographic stand-image reception plane distance) is defined as $l_c$, and a rotation angle of a radiation source arm moving the radiation source 42 is defined as θ. These are constants specific to the mammographic imaging apparatus 40 and determined from information of the mammography.

The moving distance of the radiation source may be given instead of the rotation angle θ.

These radiographic conditions are stored in the image server 32 along with the captured images of the right-eye image and the left-eye image necessary for the stereoscopic display after the mammographing, and are transmitted to the console 30 of the stereoscopic display apparatus 10 along with the captured images.

As shown in FIG. 7, the rotation axis of the radiation source arm in the mammographic imaging apparatus 40 is located at the center of the image reception plane of the detector and a panel as the detector is fixed to the radiographic stand 44 so that the center of the panel is set as the radiation source position at which the rotation angle of the radiation source arm is 0°.

When the radiation source position (L1) of 0° which serves as a reference position of the radiation source arm is represented by (X, Y)=(0, D), the radiation source-position (L2) after the rotation with an angle θ is expressed by Expression 1.

Expression 1

$$X = D \cdot \sin\theta$$
$$Y = D \cdot \cos\theta \qquad \cdots (1)$$

An image obtained by projecting a subject present at a position separated by 1 from the center of the image reception plane of the detector through the use of the radiation source 42 with a rotation angle of θ is formed at a position $x_m$ on the image reception plane calculated by Expression 2.

Expression 2

$$x_m : x_m + D\sin\theta = 1 : D\cos\theta$$
$$\Leftrightarrow 1 \cdot x_m + 1 \cdot D\sin\theta = x_m \cdot D\cos\theta$$
$$\Leftrightarrow x_m(D\cos\theta - 1) = 1 \cdot D\sin\theta \qquad \cdots (2)$$
$$\Leftrightarrow x_m = \frac{1 \cdot D\sin\theta}{D\cos\theta - 1}$$

Since the subject is necessarily present in a space interposed between the pressing plate 46 and the radiographic stand 44, nothing is present in the other space in principle. Therefore, the position which is considered to most easily view and at which no right-left parallax is present has only to be located in the space.

Therefore, by performing the calculation using the case where the subject comes in contact with the radiographic stand 44 as a case where the depth is the maximum and using the case where the subject comes in contact with the pressing plate as a case where the depth is the minimum, the range of the imaging position $x_m$ of the right-eye image and the left-eye image is determined by Expression 3.

Expression 3

$$\frac{l_c \cdot D\sin\theta}{D\cos\theta - l_c} \leqq x_m \leqq \frac{l_p \cdot D\sin\theta}{D\cos\theta - l_p} \qquad \cdots (3)$$

Here, since the range of the imaging position $x_m$ is equal to the range of the inter-image distance $x_m$ between the right-eye image and the left-eye image, the range of the inter-image distance $x_m$ between the right-eye image and the left-eye image is determined by the above-described expression. The inter-image distance $x_m$ in the first embodiment equal to the range of the imaging position is defined as a first inter-image distance $x_m$.

The range of the first inter-image distance $x_m$ is calculated in the case where one angle is 0°, that is, one (L1) of the radiographing positions is located at the reference position, as shown in FIG. 7, but the angle of the radiographing position (L1) can be generalized as $\theta_1$ and the angle of the radiographing position (L2) can be generalized $\theta_2$ ($\theta_1 \leqq \theta_2$).

In this case, the range of the first inter-image distance $x_m$ is determined by Expression 4.

Expression 4

$$\frac{l_c \cdot D\sin\theta_2 \cdot (D\cos\theta_1 - l_c) - l_c \cdot D\sin\theta_1 \cdot (D\cos\theta_2 - l_c)}{(D\cos\theta_1 - l_c)(D\cos\theta_2 - l_c)} \leqq x_m \leqq$$

$$\frac{l_p \cdot D\sin\theta_2 \cdot (D\cos\theta_1 - l_p) - l_p \cdot D\sin\theta_1 \cdot (D\cos\theta_2 - l_p)}{(D\cos\theta_1 - l_p)(D\cos\theta_2 - l_p)} \qquad \cdots (4)$$

In this embodiment, the calculation is performed on the assumption that the subject as an observation target is located in the vicinity of the center of the image reception plane.

In normal stereoscopic photography, since the capturing of an image is performed at predetermined angles (for example, 0° and 4°), the range of the first inter-image distance $x_m$ between the right-eye image and the left-eye image may be determined by preparing a table including relations between these fixed values and the pressing thicknesses and referring to the table depending on the pressing thickness during the capturing of an image.

The range of the first inter-image distance $x_m$ in which a stereoscopic view is not destroyed varies depending on the observers and it is thus difficult to uniquely determine the range. Therefore, this range is a reference not representing that the destruction of a stereoscopic view or the reversal of the depth necessarily occurs but representing that the possibility thereof is high when this range is exceeded.

Therefore, the above-mentioned table may not be calculated from measured values but may be obtained from experiences.

Hitherto, the method of calculating the range of the first inter-image distance $x_m$ between a right-eye image and a left-eye image in a stereoscopic display from the right-eye image and the left-eye image obtained through the mammography has been described, but the present invention is not limited to the mammography.

In general radiography, the same range can be obtained by measuring the thickness of a subject and using the measured thickness instead of the pressing thickness $l_p$. A table including the relationship between sex, height, weight, and the like of a subject and the thickness of the subject may be prepared, the thickness of the subject may be estimated on the basis of the body values and the table, and the estimated value may be used.

The maximum value and the minimum value of the first inter-image distance $x_m$ between the right-eye image and the left-eye image are calculated by the control unit 54 of the console 30 and are used to control the image display area 22.

The maximum value and the minimum value of the first inter-image distance $x_m$ and the changed inter-image distance are temporarily stored in the storage unit 58 of the console 30 along with the right-eye image and the left-eye image and are stored in the image server 32 via the storage unit 58.

As described above, the stereoscopic display apparatus 10 according to the present invention includes warning means that gives a warning when the inter-image distance is out of the range between the maximum and minimum values of the first inter-image distance $x_m$.

As described above, the warning means may display the effect in the text display areas 28A and 28B or may stop the changing of the inter-image distance.

The control unit 54 of the console 30 performs the calculation and the warning instruction is given via the control unit 54 and the display control unit 56 (the warning instruction is displayed).

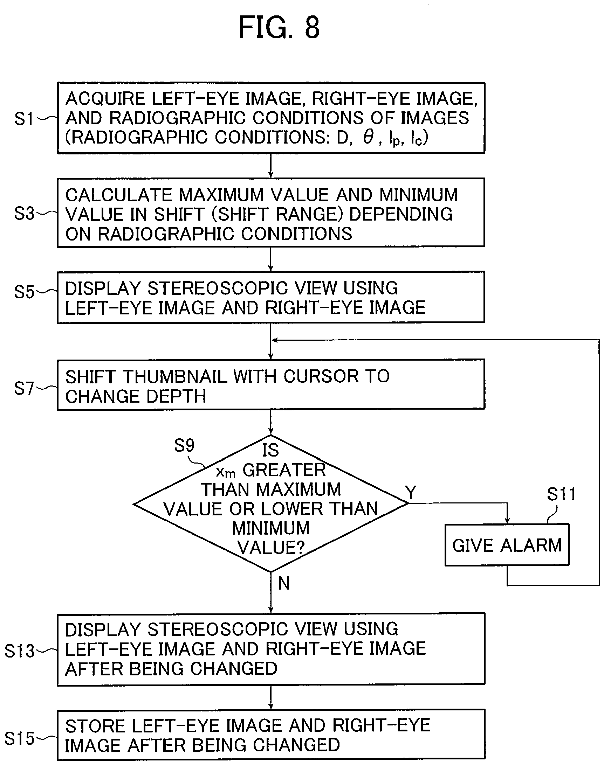

The operation of the stereoscopic display apparatus 10 according to the present invention will be described below in brief with reference to the steps in the flowchart shown in FIG. 8.

First, in step S1, a right-eye image and a left-eye image radiographed for a stereoscopic display and the radiographic conditions are acquired from the image server 32 or the like (S1).

As described above, the radiographic conditions include the radiation source-image reception plane distance (SID) D, the irradiation angle of the radiation source (the rotation angle of the radiation source arm) $\theta$, the pressing thickness (the radiographic stand-pressing plate surface distance) $l_p$, and the case thickness (the radiographic stand-image reception plane distance) $l_c$.

In step S3, the maximum and minimum values of the first inter-image distance $x_m$ between the right-eye image and the left-eye image are calculated from the radiographic conditions. In case of mammography, since a subject to be observed is considered to be present between the pressing plate and the radiographic stand, the maximum and minimum values are calculated using this range as the movable range in the depth direction (S3). In the mammography, for example, the right-eye image and the left-eye image are often fixed to a position of 0°, a position of 4°, and the like. Accordingly, when the pressing thickness $l_p$ is determined, the maximum and minimum values of the first inter-image distance $x_m$ are determined.

The maximum and minimum values of the first inter-image distance $x_m$ are calculated by the control unit 54 of the console 30 of the stereoscopic display apparatus 10. During the radiographing, the maximum and minimum values of the range allowing a stereoscopic display may be calculated in advance and the values along with the images may be stored.

When the first inter-image distance $x_m$ between the right-eye image and the left-eye image is calculated, a stereoscopic display is performed in the image display area 22 of the display unit 12 (20) by the use of the right-eye image and the left-eye image in step S5 (S5).

The thumbnail images of the right-eye image and the left-eye image are displayed in the image different display area 26 and it is thus possible to confirm by what to shift both images for the stereoscopic display.

In step S7, the thumbnail images of the right-eye image and the left-eye image in the image difference display area 26 are shifted by the use of the cursor 24 on the display unit 12 (20) to change the depth of the stereoscopic display (S7).

When the depth is changed, the shift distance between the right-eye image and the left-eye image is calculated from the shift distance of the thumbnail images and it is determined whether the first inter-image distance $x_m$ between the right-eye image and the left-eye image is in the above-mentioned range of the inter-image distance by the shift, that is, whether the first inter-image distance is out of the range between the maximum and minimum values of the first inter-image distance $x_m$ in step S9 (S9).

When it is determined that the first inter-image distance $x_m$ between the right-eye image and the left-eye image is out of the range between the calculated maximum and minimum values, a warning is given in step S11 (S11). The alarm may be visual like a blink alarm or may be auditory like a sound. The thumbnail images may be limited so as not to depart from the range of the maximum and minimum values.

When a warning is given, an operator shifts the thumbnail image through the use of the cursor and adjusts the first inter-image distance $x_m$ so as not to being out of the range between the maximum and minimum values.

When the first inter-image distance $x_m$ becomes a predetermined value by the shift of thumbnail images, at least one of the right-eye image and the left-eye image is shifted depending on the distance between the thumbnail images and the inter-image distance therebetween is used as $x_m$ after the shift, whereby the stereoscopic display is performed in step S13 (S13).

An observer confirms the stereoscopic display after the change in depth and stores the right-eye image and the left-eye image after the change in depth (the right-eye image, the left-eye image, and the distance therebetween) so as to reproduce the stereoscopic display in step S15 (S15).

Hitherto, the method of calculating the maximum and minimum values of the first inter-image distance $x_m$ between the right-eye image and the left-eye image in the stereoscopic display apparatus 10 according to the present invention has been described.

The first embodiment of the stereoscopic display apparatus according to the present invention on the premise that the stereoscopic display apparatus 10 is observed at the observation position separated by a predetermined distance from the display unit 12 of the stereoscopic display apparatus 10 has been described hitherto.

It cannot be said that the stereoscopic display of the display unit 12 is necessarily observed at the observation position as in the first embodiment. The preferable conditions for the stereoscopic display vary depending on an observer's binocular distance to be described later.

Therefore, a stereoscopic display apparatus considering observation conditions (conditions for observation: observation position and binocular distance to be described later), display conditions (an inter-pixel distance of the display apparatus), and image processing conditions (an enlargement and reduction ratio of an image) will be described as a second embodiment of the present invention.

Second Embodiment

The configuration of the stereoscopic display apparatus according to the second embodiment of the present invention is the same as the stereoscopic display apparatus 10 according to the first embodiment shown in FIGS. 1 and 2 and thus description thereof will not be repeated.

The second embodiment is different from the first embodiment, in that conditions for display (display conditions), conditions for processing image (image processing conditions), and conditions for observation (including an observer) (observation conditions) are considered in addition to the radiographic conditions when calculating the maximum and minimum values in shift of the right-eye image and the left-eye image.

An enlargement ratio (enlargement and reduction ratio) based on the pixel size of the monitor (the display unit 12) can be considered as an example of the display conditions and a display magnification based on display software can be considered as an example of the image processing conditions.

An observer's binocular distance, an observation distance (a distance between an observer and the display plane of the display unit 12 (with the second image display unit 18R disposed in front of the observer as a reference)), a right-left parallax on the display plane, an imaging plane distance (a distance to the imaging plane of a stereoscopic image), a binocular angle on the imaging plane, a binocular angle on the display plane, and the like can be considered as the observation conditions.

As shown in FIG. 9, when an observer's binocular distance is defined $D_{eye}$, an observation distance is defined as VD, a right-left parallax is defined as d', an imaging plane distance is defined as x, a binocular angle on the imaging plane is defined as $\alpha$, and a binocular angle $\beta$ on the display plane, the following expression is obtained from the binocular distance $D_{eye}$ and the observation distance VD.

Expression 5

$$\frac{D_{eye}}{2} = VD \cdot \tan\frac{\beta}{2} \qquad \cdots (5)$$

The following expression is obtained from the binocular distance $D_{eye}$ and the imaging plane distance x.

Expression 6

$$\frac{D_{eye}}{2} = x \cdot \tan\frac{\alpha}{2} \qquad \cdots (6)$$

The following expression is obtained from the right-left parallax d', the imaging plane distance x, and the observation distance VD.

Expression 7

$$\frac{d'}{2} = (x - VD) \cdot \tan\frac{\alpha}{2} \qquad \cdots (7)$$

Therefore, the following expression is obtained from Expressions 5 to 7.

Expression 8

$$d'=2 \cdot VD \cdot (\tan\frac{\beta}{2} - \tan\frac{\alpha}{2}) \qquad \cdots (8)$$

The following expressions are established by the addition theorem of tangent.

Expression 9

$$\tan(\frac{\beta}{2}-\frac{\alpha}{2}) = \frac{\tan\frac{\beta}{2}-\tan\frac{\alpha}{2}}{1+\tan\frac{\beta}{2}\cdot\tan\frac{\alpha}{2}} \qquad \cdots (9)$$

Expression 10

$$\tan\frac{\beta}{2}-\tan\frac{\alpha}{2} = \tan(\frac{\beta}{2}-\frac{\alpha}{2})\cdot(1+\tan\frac{\beta}{2}\cdot\tan\frac{\alpha}{2}) \qquad \cdots (10)$$

By substituting these expressions for Expression 8, the following expression is obtained.

Expression 11

$$d'=2 \cdot VD \cdot \{\tan(\frac{\beta}{2}-\frac{\alpha}{2})\cdot(1+\tan\frac{\alpha}{2}\cdot\tan\frac{\beta}{2})\} \qquad \cdots (11)$$

Here, since the parallax angle $\phi_2$ is expressed by $\phi_2=\beta-\alpha$, Expression 11 is changed to the following expression.

Expression 12

$$d'=2 \cdot VD \cdot \tan\frac{\phi_2}{2} \cdot \{1+\tan(\frac{\beta-\phi_2}{2})\cdot\tan\frac{\beta}{2}\} \qquad \cdots (12)$$

By applying the addition theorem of tangent to Expression 12, the following expression is obtained.

Expression 13

$$d'=2 \cdot VD \cdot \tan\frac{\phi_2}{2} \cdot \left\{1+\tan\frac{\beta}{2}\cdot\frac{\tan\frac{\beta}{2}-\tan\frac{\phi_2}{2}}{\tan\frac{\beta}{2}\cdot\tan\frac{\phi_2}{2}+1}\right\} \qquad \cdots (13)$$

The following expression is established by Expression 5.

Expression 14

$$\tan \frac{\beta}{2} = \frac{D_{eye}}{2 \cdot VD} \qquad \cdots (14)$$

By replacing $\tan(\beta/2)$ in Expression 12 with Expression 14, the following expressions can be obtained.

Expression 15

$$d' = 2 \cdot VD \cdot \tan\frac{\phi_2}{2} \cdot \left\{ 1 + \frac{D_{eye}}{2 \cdot VD} \cdot \frac{\dfrac{D_{eye}}{2 \cdot VD} - \tan\frac{\phi_2}{2}}{\dfrac{D_{eye}}{2 \cdot VD} \cdot \tan\frac{\phi_2}{2} + 1} \right\}$$

$$\Leftrightarrow d' = 2 \cdot VD \cdot \tan\frac{\phi_2}{2} + D_{eye} \cdot \tan\frac{\phi_2}{2} \cdot \frac{\dfrac{D_{eye}}{2 \cdot VD} - \tan\frac{\phi_2}{2}}{\dfrac{D_{eye}}{2 \cdot VD} \cdot \tan\frac{\phi_2}{2} + 1}$$

$$\Leftrightarrow d' = 2 \cdot VD \cdot \tan\frac{\phi_2}{2} + D_{eye} \cdot \tan\frac{\phi_2}{2} \cdot \frac{D_{eye} - \tan\frac{\phi_2}{2} \cdot 2 \cdot VD}{D_{eye} \cdot \tan\frac{\phi_2}{2} + 2 \cdot VD}$$

$$\Leftrightarrow d' = \frac{2 \cdot VD \cdot \tan\frac{\phi_2}{2} \cdot (D_{eye} \cdot \tan\frac{\phi_2}{2} + 2 \cdot VD)}{D_{eye} \cdot \tan\frac{\phi_2}{2} + 2 \cdot VD} + \frac{D_{eye}^2 \cdot \tan\frac{\phi_2}{2} - D_{eye} \cdot \tan^2\frac{\phi_2}{2} \cdot 2 \cdot VD}{D_{eye} \cdot \tan\frac{\phi_2}{2} + 2 \cdot VD}$$

$$\Leftrightarrow d' = \frac{D_{eye} \cdot \tan^2\frac{\phi_2}{2} \cdot 2 \cdot VD + 4 \cdot VD^2 \cdot \tan\frac{\phi_2}{2}}{D_{eye} \cdot \tan\frac{\phi_2}{2} + 2 \cdot VD} + \frac{D_{eye}^2 \cdot \tan\frac{\phi_2}{2} - D_{eye} \cdot \tan^2\frac{\phi_2}{2} \cdot 2 \cdot VD}{D_{eye} \cdot \tan\frac{\phi_2}{2} + 2 \cdot VD}$$

$$\Leftrightarrow d' = \frac{D_{eye}^2 \cdot \tan\frac{\phi_2}{2} + 4 \cdot VD^2 \cdot \tan\frac{\phi_2}{2}}{D_{eye} \cdot \tan\frac{\phi_2}{2} + 2 \cdot VD}$$

$$\Leftrightarrow d' = \frac{\tan\frac{\phi_2}{2} \cdot (D_{eye}^2 + 4VD^2)}{2VD + D_{eye} \cdot \tan\frac{\phi_2}{2}} \qquad \cdots (15)$$

The position gap on the image reception plane during radiographing is defined as $x_m$ (the first inter-image distance in the first embodiment, that is, an imaging position), the radiation source-image reception plane distance (SID) is defined as D, the distance from the center of the image reception plane of the detector to a subject (position of interest) is defined as 1 (radiographic stand thickness (radiographic stand-image reception plane distance) $l_c \leq l \leq$ pressing thickness (radiographic stand-pressing plate surface distance) $l_p$ in the first embodiment), and the rotation angle difference of the radiation source arm is defined as $\psi_1$ (bulb oscillation angle $\psi_1$ in the first embodiment: $\psi_1 = \theta_2 - \theta_1$).

Then, the inter-bulb distance during radiographing is expressed by $2 \cdot D \cdot \tan(\psi_1/2)$ and the distance from the bulb to the pressing plate is expressed by D-1.

When it is assumed that a moving plane of the bulb is substantially parallel to the image reception plane (In general, when a right-eye image and a left-eye image used for a stereoscopic display are acquired, the rotation angle difference $\psi_1$ of the radiation source arm is so small that both may be considered to be substantially parallel. This is intended to prevent destruction of a stereoscopic display.), the following expression is established.

Expression 16

$$x_m : 2 \cdot D \cdot \tan \frac{\phi_1}{2} = 1 : D-1 \qquad \cdots (16)$$

Accordingly, the position gap $x_m$ is expressed by the following expression.

Expression 17

$$x_m = \frac{2 \cdot D \cdot \tan \dfrac{\phi_1}{2} \cdot 1}{D-1} \qquad \cdots (17)$$

The right-left parallax d' on the display plane is an appearing size of an image displayed on the monitor (the display unit 12) and the position gap $x_m$ of image data depends on the gap of imaging device. Therefore, it is necessary to match the size of the imaging device and the size of the display device with each other. When the inter-pixel distance of the imaging device is $d_i$ and the inter-pixel distance of the display apparatus $d_m$, the gap during displaying can be set to the same scale as the gap during imaging by setting the size of an image on the monitor to the $d_i/d_m$ magnification ($=m_1$).

When an image enlarged at an $m_2$ magnification by the image processing unit 52 is watched, d' is a magnitude which can be obtained by enlarging the original image and it is thus necessary to match d' with the distance at an enlargement ratio of 1 magnification (at the same magnification).

When the enlargement ratio of the pixel size in the monitor (the display unit 12) as the display condition is defined as $m_1$ and the display magnification by display software as the image processing condition is defined as $m_2$, the second inter-image distance (image shift distance) $\Delta d$ between a right-eye image and a left-eye image in the second embodiment is expressed as follows.

Expression 18

$$\Delta d = \frac{d'}{m_1 m_2} - x_m \qquad \cdots (18)$$

Therefore, it is possible to calculate the range of the second inter-image distance $\Delta d$ in which the right eye and the left eye can be shifted, as in the first embodiment, using Expressions 15, 17, and 18 and the distance l ($l_c \leq l \leq l_p$) between the center of the image reception plane of the detector and a subject (position of interest).

The range of the second inter-image distance $\Delta d$ is expressed by the following expression.

Expression 19

$$\frac{\tan\frac{\phi_2}{2} \cdot (D_{eye}^2 + 4VD^2)}{m_1 m_2 (2VD + D_{eye} \cdot \tan\frac{\phi_2}{2})} - \frac{2 \cdot D \cdot \tan\frac{\phi_1}{2} \cdot l_p}{D - l_p} \leqq \Delta d \leqq$$

$$\frac{\tan\frac{\phi_2}{2} \cdot (D_{eye}^2 + 4VD^2)}{m_1 m_2 (2VD + D_{eye} \cdot \tan\frac{\phi_2}{2})} - \frac{2 \cdot D \cdot \tan\frac{\phi_1}{2} \cdot l_c}{D - l_c} \qquad \cdots (19)$$

In this way, the stereoscopic display apparatus 10 according to the second embodiment of the present invention calculates the inter-image distance between a right-eye image and a left-eye image in consideration of the conditions for radiographing and observation and performs a stereoscopic display on the basis of the inter-image distance, and thus can perform a stereoscopic display which can be observed more easily than in the first embodiment and which considers the situation of radiographing and the situation of observation.

In the stereoscopic display apparatus 10 according to the second embodiment, the radiographic conditions, the observation conditions, the display conditions, and the image processing conditions as well as the image data of the right-eye image and the left-eye image and the inter-image distance may be stored in the storage unit 58 of the console 30 and may be used again to refer to the stereoscopic display.

Particularly, the observation conditions vary depending on the purpose of the stereoscopic display or the observer's binocular distance and are thus stored in correlation with the purpose or the observer information. When the image data of the right-eye image and the left-eye image and the inter-image distance therebetween are read again by another observer or for another purpose, the inter-image distance may be corrected by the image processing unit 52, the control unit 54, and the display control unit 56 on the basis of another observer's observation conditions (mainly the binocular distance).

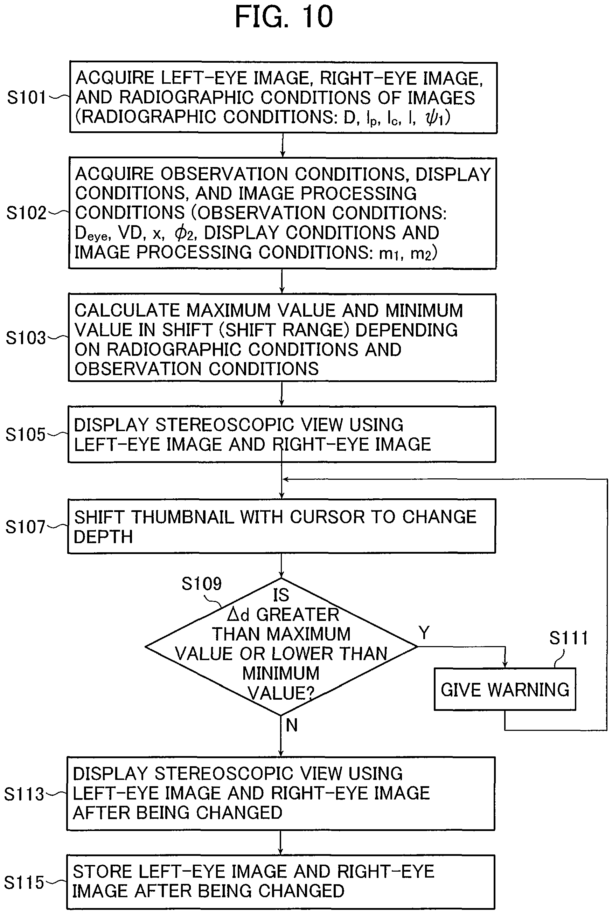

The operation of the stereoscopic display apparatus 10 according to the second embodiment of the invention will be described below in brief with reference to the steps of the flowchart shown in FIG. 10.

First, similarly to the flow in the first embodiment, a right-eye image and a left-eye image radiographed for a stereoscopic display and the radiographic conditions thereof are acquired from the image server 32 or the like in step S101 (S101).

As in the first embodiment, the radiographic conditions include the radiation source-image reception plane distance (SID) D, the irradiation angles $\theta_1$ and $\theta_2$ of the radiation source (the rotation angle of the radiation source arm, that is, the bulb oscillation angle $\psi_1 = \theta_2 - \theta_1$, $\theta_2 \geq \theta_1$), the pressing thickness (the radiographic stand-pressing plate surface distance) $l_p$, and the case thickness (the radiographic stand-image reception plane distance) $l_c$.

In step S102, as described above, an observer's binocular distance $D_{eye}$, an observation distance VD, a right-left parallax d' on the display plane, an imaging plane distance x, a binocular angle $\alpha$ on the imaging plane, a binocular angle $\beta$ on the display plane, and the like are input as the observation conditions through the use of the operation input unit 16. Predetermined values may be input in advance as the observation conditions. Similarly to the observation conditions, the conditions used depending on the screen size of the display unit 12 or the magnitude of the image data are acquired as the display conditions (the enlargement ratio $m_1$ based on the pixel size of the monitor) and the image processing conditions (the display magnification $m_2$ of display software) (S102).

In step S103, the maximum and minimum values of the second inter-image distance (image shift distance) $\Delta d$ between the right-eye image and the left-eye image are calculated from the radiographic conditions, the observation conditions, and the like.

As described above, when the enlargement ratio based on the pixel size of the monitor is defined as $m_1$ and the display magnification of display software is defined as $m_2$, the second inter-image distance $\Delta d$ is calculated by Expression 17.

As described above, in case of mammography, since a subject to be observed is considered to be present between the pressing plate and the radiographic stand, the maximum and minimum values are calculated using this range as the movable range in the depth direction (S103). In the mammography, for example, the right-eye image and the left-eye image are often fixed to a position of 0°, a position of 4°, and the like. Accordingly, when the pressing thickness $l_p$ is determined, the maximum and minimum values of the second inter-image distance $\Delta d$ are determined.

The maximum and minimum values of the second inter-image distance $\Delta d$ are calculated by the control unit 54 of the console 30 of the stereoscopic display apparatus 10. During the radiographing, the maximum and minimum values of the range allowing a stereoscopic display may be calculated in advance and the values along with the images may be stored.

As in the first embodiment, when the second inter-image distance $\Delta d$ between the right-eye image and the left-eye image is calculated, a stereoscopic display is performed in the image display area 22 of the display unit 12 (20) by the use of the right-eye image and the left-eye image in step S105 (S105).

The thumbnail images of the right-eye image and the left-eye image are displayed in the image difference display area 26 and it is thus possible to confirm by what to shift both images for the stereoscopic display.

Similarly, in step S107, the thumbnail images of the right-eye image and the left-eye image in the image difference display area 26 are shifted by the use of the cursor 24 on the display unit 12 (20) to change the depth of the stereoscopic display (S107).

When the depth is changed, the shift distance between the right-eye image and the left-eye image is calculated from the shift distance of the thumbnail images and it is determined whether the second inter-image distance $\Delta d$ between the right-eye image and the left-eye image is in the above-mentioned range of the inter-image distance by the shift, that is, whether the second inter-image distance $\Delta d$ is out of the range between the maximum and minimum values of the second inter-image distance $\Delta d$ in step S109 (S109).

When it is determined that the second inter-image distance $\Delta d$ between the right-eye image and the left-eye image is out of the range between the calculated maximum and minimum values, a warning is given in step S111 (S111) as in the first embodiment. The alarm may be visual like a blink alarm or may be auditory like a sound. The thumbnail images may be limited so as not to depart from the range of the maximum and minimum values.

When a warning is given, an operator shifts the thumbnail image through the use of the cursor and adjusts the second inter-image distance $\Delta d$ so as not to being out of the range between the maximum and minimum values.

When the second inter-image distance $\Delta d$ becomes a predetermined value by the shift of thumbnail images, at least one of the right-eye image and the left-eye image is shifted depending on the distance between the thumbnail images and the inter-image distance therebetween is used as $\Delta d$ after the shift, whereby the stereoscopic display is performed in step S113 (S113).

An observer confirms the stereoscopic display after the change in depth and stores the right-eye image and the left-eye image after the change in depth (image data of the right-eye image and the left-eye image, the radiographic conditions, the observation conditions, and the like) so as to reproduce the stereoscopic display in step S115 (S115).

The second inter-image distance $\Delta d$ and the observation conditions are stored along with the image data of the right-eye image and the left-eye image. Accordingly, even when an observer is changed, it is possible to perform a desired stereoscopic display on th the basis of the stored image data of the right-eye image and the left-eye image by changing the observation conditions and re-calculating the second inter-image distance $\Delta d$.

Hitherto, the stereoscopic display apparatus according to the second embodiment of the present invention considering the observation conditions, the display conditions, and the image processing conditions in addition to the radiographic conditions has been described.

In the first embodiment and the second embodiment, the inter-image distance (which is $x_m$ in the first embodiment and $\Delta d$ in the second embodiment and which includes the first inter-image distance $x_m$ and the second inter-image distance $\Delta d$) is manually changed (by shifting the thumbnail images through the use of the cursor 24). The changing means may have an automatic control function and the inter-image distances $x_m$ and $\Delta d$ may be automatically changed by the console 30 on the basis of the radiographic conditions and the like (the observation conditions, the display conditions, and the image processing conditions in addition to the radiographic conditions in the second embodiment).

For example, the inter-image distances $x_m$ and $\Delta d$ may be changed so as to is the deepest in the depth direction without destroying the stereoscopic display on the basis of the pressing thickness $l_p$ and the irradiation angles $\theta_1$ and $\theta_2$ of the radiation source (the bulb oscillation angle $\psi_1$) and may be changed to the center of the controllable range so as for an operator to easily readjust the inter-image distances. The inter-image distances may be changed so as to be the shortest in the depth direction on the premise that the operator changes the inter-image distances.

The changing means may have an automatic control function and thus the inter-image distances $x_m$ and $\Delta d$ may be automatically changed on the basis of the inter-image distances of another stereoscopic display stored in the image server 32 by the console 30.

For example, when it is intended to compare stereoscopic displays, the inter-image distances $x_m$ and $\Delta d$ of the present stereoscopic display are determined on the basis of the inter-image distances of another stereoscopic display as a comparison target stored in the image server 32.

Blanks of a right-eye image and a left-eye image generated when the inter-image distances are changed by the changing means may be painted with a predetermined color during the stereoscopic display. For example, the blanks may be painted with black. By painting the blanks with black, it is possible to prevent erroneous diagnosis in radiographic image diagnosis and to cause an image area as a transmitted image to be visible well.

When the inter-image distances are changed by the changing means, it is preferable that it is stored what observer changes the inter-image distances by the use of what stereoscopic display apparatus (the display unit 12). By correcting the stereoscopic display on the basis of the observation conditions (binocular distance and observation distance) different depending on observers when the observer is changed and correcting the stereoscopic display on the basis of the inter-image distances (display conditions (enlargement and reduction ratio) and image processing conditions (display magnification)) different depending on the display apparatuses when the stereoscopic display apparatus is changed, it is possible to perform a stereoscopic display optimal for other observers only by fine adjustment.

The stereoscopic display apparatus 10 according to the present invention employs a synthesis display system using polarized light and a half mirror, but the present invention is not limited to this system and may be applied to stereoscopic display apparatuses employing other display systems.

While the stereoscopic display apparatus according to the embodiment of the invention has been described above in detail, the invention is not limited to the embodiment but may be improved or modified in various forms without departing from the concept of the invention.

**Claims**

1. A stereoscopic display apparatus that performs a stereoscopic display using a right-eye image and a left-eye image acquired by radiographing a subject plural times while changing an incidence angle of radiation on the subject, comprising:

   a display unit that stereoscopically displays the right-eye image and the left-eye image; and
   changing means that shifts at least one of the right-eye image and the left-eye image displayed on the display unit to change an inter-image distance being a distance between the right-eye image and the left-eye image in a direction parallel to a straight line connecting the right eye and the left eye of an observer.

2. The stereoscopic display apparatus according to claim 1, wherein the inter-image distance includes a first inter-image distance and a second inter-image distance,
   the first inter-image distance being calculated on the basis of radiographic conditions of the right-eye image and the left-eye image, and
   the second inter-image distance being calculated on the basis of the radiographic conditions, and observation conditions, display conditions and image processing conditions of the stereoscopic display, and
   wherein a range of the inter-image distance which can be changed by the changing means is calculated based on the first inter-image distance and the second inter-image distance.

3. The stereoscopic display apparatus according to claim 1 or 2, further comprising warning means that gives a warning when the inter-image distance is out of the range of the inter-image distance by the changing means.

4. The stereoscopic display apparatus according to claim 3, wherein the warning of the warning means includes at least one of stopping the changing of the inter-image distance and displaying the purport thereof.

5. The stereoscopic display apparatus according to any one of claims 2 to 4, wherein when the radiographic conditions includes a distance D between a radiation source and an image reception plane, irradiation angles of a radiation source $\theta_1$ and $\theta_2$ being rotation angles of a radiation source arm, $\theta_2$ being greater than or equal to $\theta_1$, a pressing thickness $l_p$ being a distance between a radiographic stand and a pressing plate surface, and a case thickness $l_c$ being a distance between the radiographic stand and the image reception plane, the range of the first inter-image distance $x_m$ is expressed by the following formula:

$$\frac{l_c \cdot D\sin\theta_2 \cdot (D\cos\theta_1 - l_c) - l_c \cdot D\sin\theta_1 \cdot (D\cos\theta_2 - l_c)}{(D\cos\theta_1 - l_c)(D\cos\theta_2 - l_c)} \leqq x_m \leqq$$

$$\frac{l_p \cdot D\sin\theta_2 \cdot (D\cos\theta_1 - l_p) - l_p \cdot D\sin\theta_1 \cdot (D\cos\theta_2 - l_p)}{(D\cos\theta_1 - l_p)(D\cos\theta_2 - l_p)} \quad .$$

6. The stereoscopic display apparatus according to claim 5, wherein when the observation conditions include an observation distance VD which is a distance between the display unit and the observer, a binocular distance $D_{eye}$, an imaging plane distance x, a parallax angle $\phi_2$ being a difference between a binocular angle in the display unit and a binocular angle at an imaging position, and a right-left parallax d' on the display unit, the right-left parallax d' is expressed by the following formula:

$$d' = \frac{\tan\frac{\phi_2}{2} \cdot (D_{eye}^2 + 4VD^2)}{2VD + D_{eye} \cdot \tan\frac{\phi_2}{2}} \quad .$$

7. The stereoscopic display apparatus according to claim 6, wherein the display conditions include an enlargement and reduction ratio based on an inter-pixel distance being a pixel size of the display unit.

8. The stereoscopic display apparatus according to claim 6 or 7, wherein the image processing conditions include a display magnification of the right-eye image and the left-eye image.

9. The stereoscopic display apparatus according to claim 8, wherein when the display conditions include an enlargement and reduction ratio $m_1$ and the image processing conditions include a display magnification $m_2$, the range of the second inter-image distance $\Delta d$ is expressed by

$$\Delta d = \frac{d'}{m_1 m_2} - x_m \quad ,$$

and
wherein when a difference $\theta_2 - \theta_1$ in the rotation angles $\theta_1$ and $\theta_2$ of the radiation source arm is $\psi_1$, the second inter-image distance $\Delta d$ is expressed by the following formula:

$$\frac{\tan\frac{\phi_2}{2} \cdot (D_{eye}^2 + 4VD^2)}{m_1 m_2 (2VD + D_{eye} \cdot \tan\frac{\phi_2}{2})} - \frac{2 \cdot D \cdot \tan\frac{\phi_1}{2} \cdot l_p}{D - l_p} \leqq \Delta d \leqq$$

$$\frac{\tan\frac{\phi_2}{2} \cdot (D_{eye}^2 + 4VD^2)}{m_1 m_2 (2VD + D_{eye} \cdot \tan\frac{\phi_2}{2})} - \frac{2 \cdot D \cdot \tan\frac{\phi_1}{2} \cdot l_c}{D - l_c} \quad .$$

10. The stereoscopic display apparatus according to any one of claims 1 to 9, further comprising an image difference display area in which the inter-image distance between the right-eye image and the left-eye image is displayed by the use of thumbnail images,
wherein the changing means changes the distance between the thumbnail images displayed in the image difference display area to adjust the depth of the stereoscopic display.

11. The stereoscopic display apparatus according to any one of claims 1 to 10, wherein the changing means has an automatic control function, and
wherein the automatic control function is to automatically change the inter-image distance depending on the radiographic conditions.

12. The stereoscopic display apparatus according to claim 11, wherein the automatic control function is to change the inter-image distance to any one of a maximum value, a median value, and a minimum value of the range of the changeable inter-image distance.

13. The stereoscopic display apparatus according to any one of claims 1 to 10, further comprising an image server that stores a plurality of the inter-image distances between right-eye images and left-eye images which can be stereoscopically displayed,
wherein the changing means has an automatic control function, and
wherein the automatic control function is to automatically change the inter-image distance of the present stereoscopic display depending on the inter-image distance of the other stereoscopic displays.

14. The stereoscopic display apparatus according to any one of claims 1 to 13, wherein blanks of the right-eye image and the left-eye image formed by causing the changing means to change the inter-image distance are painted with black.

# FIG. 1

# FIG. 2

FIG. 2 block diagram showing: IMAGE SERVER (32) connected to IMAGE ACQUIRING UNIT (50) with CTL input; IMAGE ACQUIRING UNIT connected to IMAGE PROCESSING UNIT (52) with CTL input; IMAGE PROCESSING UNIT connected to DISPLAY CONTROL UNIT (56) with CTL input; DISPLAY CONTROL UNIT (56) connected to FIRST IMAGE DISPLAY UNIT (18L) and SECOND IMAGE DISPLAY UNIT (18R); OPERATION INPUT UNIT (16) connected to CONTROL UNIT (54) with CTL output; STORAGE UNIT (58) with CTL input. Outer box labeled 30, system labeled 12.

EP 2 506 586 A2

# FIG. 3

# FIG. 4

FIG. 5A

LEFT EYE    RIGHT EYE

FIG. 5B

LEFT-EYE    RIGHT-EYE
IMAGE       IMAGE

FIG. 6A

LEFT-EYE    RIGHT-EYE
IMAGE       IMAGE

FIG. 6B

LEFT EYE    RIGHT EYE

# FIG. 7

# FIG. 9

# FIG. 8

S1 — ACQUIRE LEFT-EYE IMAGE, RIGHT-EYE IMAGE, AND RADIOGRAPHIC CONDITIONS OF IMAGES (RADIOGRAPHIC CONDITIONS: D, $\theta$, $I_p$, $I_c$)

S3 — CALCULATE MAXIMUM VALUE AND MINIMUM VALUE IN SHIFT (SHIFT RANGE) DEPENDING ON RADIOGRAPHIC CONDITIONS

S5 — DISPLAY STEREOSCOPIC VIEW USING LEFT-EYE IMAGE AND RIGHT-EYE IMAGE

S7 — SHIFT THUMBNAIL WITH CURSOR TO CHANGE DEPTH

S9 — IS $x_m$ GREATER THAN MAXIMUM VALUE OR LOWER THAN MINIMUM VALUE?

Y

S11 — GIVE ALARM

N

S13 — DISPLAY STEREOSCOPIC VIEW USING LEFT-EYE IMAGE AND RIGHT-EYE IMAGE AFTER BEING CHANGED

S15 — STORE LEFT-EYE IMAGE AND RIGHT-EYE IMAGE AFTER BEING CHANGED

# FIG. 10

S101 — ACQUIRE LEFT-EYE IMAGE, RIGHT-EYE IMAGE, AND RADIOGRAPHIC CONDITIONS OF IMAGES (RADIOGRAPHIC CONDITIONS: D, $I_p$, $I_c$, I, $\psi_1$)

S102 — ACQUIRE OBSERVATION CONDITIONS, DISPLAY CONDITIONS, AND IMAGE PROCESSING CONDITIONS (OBSERVATION CONDITIONS: $D_{eye}$, VD, x, $\phi_2$, DISPLAY CONDITIONS AND IMAGE PROCESSING CONDITIONS: $m_1$, $m_2$)

S103 — CALCULATE MAXIMUM VALUE AND MINIMUM VALUE IN SHIFT (SHIFT RANGE) DEPENDING ON RADIOGRAPHIC CONDITIONS AND OBSERVATION CONDITIONS

S105 — DISPLAY STEREOSCOPIC VIEW USING LEFT-EYE IMAGE AND RIGHT-EYE IMAGE

S107 — SHIFT THUMBNAIL WITH CURSOR TO CHANGE DEPTH

S109 — IS $\Delta$d GREATER THAN MAXIMUM VALUE OR LOWER THAN MINIMUM VALUE?

Y → S111 — GIVE WARNING

N

S113 — DISPLAY STEREOSCOPIC VIEW USING LEFT-EYE IMAGE AND RIGHT-EYE IMAGE AFTER BEING CHANGED

S115 — STORE LEFT-EYE IMAGE AND RIGHT-EYE IMAGE AFTER BEING CHANGED

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010200787 A

- JP 3780217 B